# NOUVEAU FASCICULE DE BREVET EUROPEEN

(11) **EP 3 119 529 B2**
(45) Date de publication et mention de la décision concernant l'opposition: **04.06.2025**
(45) Mention de la délivrance du brevet: 11.11.2020
(21) Numéro de dépôt: 15715351.1
(22) Date de dépôt: 19.03.2015
(51) Int. Cl.: B05B 11/00, B05B 11/02, B05B 11/04, B05B 15/52, B65D 47/20, B65D 47/40, B05B 15/40, B65D 83/34

(54) **DISPOSITIF DE DISTRIBUTION DE LIQUIDE COMPRENANT UN CAPUCHON DE PROTECTION**
FLÜSSIGAUSGABEVORRICHTUNG MIT EINER SCHUTZKAPPE
LIQUID-DISPENSING DEVICE COMPRISING A PROTECTIVE CAP

(30) Priorité: 20.03.2014 FR 1452341
(43) Date de publication de la demande: 25.01.2017
(73) Titulaire: Nemera La Verpilliere, F-38290 La Verpilliere (FR)
(72) Inventeur: DECOCK, Thierry, F-69002 Lyon (FR); PAINCHAUD, Gaëtan, F-69340 Francheville (FR); QUAGLIA, Benjamin, F-69007 Lyon (FR)
(74) Mandataire: Lavoix
(86) Numéro de dépôt international: PCT/FR2015/050680
(87) Numéro de publication internationale: WO 2015/140473

(56) Documents cités:
- WO-A1-2012/100013
- FR-A1- 2 988 015
- JP-A- H09 150 855
- US-A- 4 936 700

## Description

La présente invention concernele domaine technique de la distribution de liquide ophtalmique.

On connaît dans l'état de la technique, par exemple dans le document FR 2 988 015, un dispositif de distribution de liquide comportant un flacon, un embout de distribution muni d'une ouverture de distribution de liquide et un capuchon amovible de protection de cette ouverture. Le capuchon de protection comprend un tampon d'absorption, réalisé de manière à ce que du liquide résiduel soit absorbé par le tampon, permettant ainsi de limiter l'administration ultérieure de liquide résiduel contaminé.

Il se trouve que, au cours du procédé de montage du tampon d'absorption à l'intérieur du capuchon de protection, des particules provenant du tampon peuvent être générées, notamment par les frottements apparaissant lors de la manipulation du tampon sur le capuchon de protection. Or, ces particules peuvent être à l'origine de la contamination du produit, ou encore de l'administration de particules dans l'œil. En outre, ces particules sont susceptibles de s'accumuler dans différentes parties du dispositif, par exemple en se déposant sur le tampon d'absorption, à l'intérieur du dispositif, au risque de créer un disfonctionnement d'une valve anti-retour, ou encore dans une salle blanche d'assemblage du dispositif, salle dans laquelle le taux de particules présentes à l'intérieur fait l'objet de réglementations et de contrôles.

La présente invention a notamment pour but de fournir un dispositif de distribution de liquide empêchant davantage la contamination du produit distribué.

A cet effet, l'invention a pour objet un dispositif de distribution de liquide selon la revendication 1.

Ainsi, le tampon d'absorption est fixé sur le capuchon de protection par l'intermédiaire du support. On propose de fixer le tampon sur un support, ce qui permet de réduire, voire de limiter totalement, la génération de particules qui pourrait survenir lors du montage du tampon dans le capuchon de protection. En effet, comme le tampon est généralement réalisé dans un matériau relativement friable, le fait de le fixer sur un support plutôt que directement sur le capuchon de protection a pour conséquence que, dans le cas où des particules seraient générées lors de la fixation du tampon d'absorption dans le support, ces particules peuvent être ensuite enlevées, et ne se retrouvent pas coincées à l'intérieur du capuchon de protection. Par ailleurs, comme le support est réalisé dans un matériau plus résistant que le tampon d'absorption, les forces qui sont exercées par le capuchon de protection sur le tampon d'absorption au cours de l'assemblage sont appliquées sur le support, ce qui génère moins de particules, car il n'y a plus ou quasiment plus de frottements entre le tampon d'absorption et le capuchon de protection lors de l'assemblage. En conséquence, on obtient un dispositif qui limite la quantité de particules susceptibles de contaminer le produit à administrer et qui réduit le risque d'administrer involontairement dans l'œil des particules pouvant être nocives, gênantes et/ou permettant de respecter certaines contraintes règlementaires.

On comprend donc que le bord latéral du tampon d'absorption est espacé de contours de centrage du tampon d'absorption dans le capuchon de protection, si bien que les contacts et/ou frottements sont évités entre le tampon d'absorption et les contours de centrage, donc entre le tampon d'absorption et le capuchon de protection. Il n'y a donc pas ou peu de formation de particules lorsque l'on rapporte le support muni du tampon d'absorption dans le capuchon de protection.

Les moyens d'espacement du bord latéral du tampon d'absorption sont portés par le support, par exemple par les parois latérales du support.

On entend par « matériau plus résistant que le tampon d'absorption » un matériau plus cohésif que celui du tampon d'absorption, c'est-à-dire que les forces d'interaction au sein de la matière du support sont plus fortes que celles au sein de la matière du tampon d'absorption, en particulier les forces d'interaction électromagnétique. Comme le support est réalisé dans un matériau plus résistant, il présente une friabilité réduite et une résistance accrue aux frottements, permettant donc de limiter la génération de particules. Par exemple, le support est réalisé en matière plastique telle que le polyéthylène (PE), et le tampon d'absorption est réalisé dans un matériau comprenant au moins une partie hydrophile, permettant le drainage de liquide, c'est-à-dire capable d'absorber du liquide, voire de le laisser s'évaporer si un chemin d'évaporation est ouvert.

Le support est fixé dans le capuchon de protection par assemblage mécanique, par exemple par serrage ou par encliquetage, éventuellement par l'intermédiaire d'un ou plusieurs reliefs ou creux, continus ou non, tels qu'une nervure ou un plot, ménagés sur le capuchon de protection et/ou le support. Ainsi, après insertion du support en forçant à l'intérieur du capuchon de protection et déformation élastique du support au passage du ou des relief(s), le relief et/ou le creux peuvent former une butée mécanique du support dans le capuchon de protection. Il est également possible de prévoir un relief de géométrie différente capable d'exercer une fonction de blocage du support par rapport au capuchon de protection. Il est également possible de réaliser un collage du support dans le capuchon de protection. Ainsi, l'ensemble pourra être manipulé et assemblé avec d'autres pièces en limitant au maximum un éventuel désassemblage du capuchon de protection et du support sur lequel le tampon d'absorption est fixé.

Le dispositif présenté ci-dessus peut en outre comporter l'une ou plusieurs des caractéristiques suivantes, prises seules ou en combinaison.
- Le support est réalisé en matière plastique, telle que du polyéthylène. La matière plastique offre une meilleure résistance au support qu'au tampon d'absorption, de façon à limiter la génération de particules sous l'effet des frottements qui pourraient apparaître au cours du montage.
- Le support a une forme annulaire. Cette forme annulaire permet de limiter les frottements sur tout le pourtour du tampon d'absorption.
- Le support s'étend sur toute la hauteur du tampon d'absorption. Ceci permet ainsi de restreindre ou supprimer toute partie du tampon d'absorption en contact avec le capuchon de protection.
- Le tampon d'absorption de liquide résiduel est fixé sur le support par surmoulage. Le surmoulage du tampon d'absorption sur le support ou du support sur le tampon d'absorption présente l'avantage de ne pas générer du tout de particules au cours de l'assemblage, et permet en outre d'assurer une unicité à l'ensemble comprenant le support et le tampon d'absorption, pour permettre une bonne cohésion entre ces deux parties.
- Le tampon d'absorption est fixé sur le support, par serrage, encliquetage, collage ou posé sur le support de façon à ce que le tampon d'absorption soit retenu sur le support. Un tel assemblage est particulièrement simple à mettre en oeuvre.
- Le tampon d'absorption est réalisé dans un matériau poreux. Ainsi, le matériau poreux permet de disposer de propriétés d'absorption satisfaisantes, en particulier des propriétés hydrophiles, utiles à l'absorption du liquide résiduel par le tampon d'absorption, de façon à obtenir une absorption quasi-complète ou complète du liquide résiduel par le tampon. Il peut, en outre, permettre une protection contre d'éventuelles contaminations microbiennes grâce à l'absorption, drainage et séchage du liquide résiduel due à la nature poreuse du matériau.
- Le tampon d'absorption comprend des matériaux tissés ou non tissés. Il peut par exemple comprendre du coton hydrophile, du polyéthylène (PE) ayant subi un traitement hydrophile, du polyéthylène téréphtalate (PET) ayant subi un traitement hydrophile, une mousse de poly(acétate de vinyle) (PVA) ou encore un mélange de plusieurs matériaux hydrophiles.
- Le tampon d'absorption est réalisé dans un matériau comprenant de l'éthylène acétate de vinyle (EVA). L'utilisation de ce matériau pour la réalisation du tampon d'absorption est particulièrement avantageuse car elle permet de limiter la formation de particules. En effet, la structure de l'EVA, sous forme de mousse, a une meilleure cohésion que celle du tampon d'absorption, tout en maintenant les propriétés absorbantes de celui-ci.
- Le tampon d'absorption est un tampon multicouche. Les couches sont de préférence empilées l'une sur l'autre axialement. En particulier, le tampon d'absorption peut comprendre au moins deux matériaux absorbants distincts, disposés l'un au-dessus de l'autre, le matériau en aval étant plus hydrophile que le matériau en amont. Ainsi, le liquide est aspiré par le matériau le plus hydrophile et le liquide résiduel ne stagne pas dans le matériau le moins hydrophile disposé à proximité de l'ouverture de distribution. La goutte résiduelle est ainsi entièrement absorbée grâce à la proximité du tampon d'absorption. Le matériau en amont comprend avantageusement un matériau hydrophobe. Il empêche donc le liquide absorbé par le matériau le plus hydrophile de retourner vers l'ouverture de distribution. Le tampon d'absorption peut contenir en outre, sur au moins une de ses couches, un agent antimicrobien qui peut être de type désinfectant ou bactériostatique. L'avantage d'un tel tampon d'absorption est une très grande efficacité microbienne, sans contact de cet agent antimicrobien avec le liquide délivré.
- La surface du tampon d'absorption en contact avec l'air peut être augmentée, notamment pour accélérer la vitesse de séchage du tampon, en particulier lorsqu'un tampon de basse absorption est utilisé.
- Le tampon d'absorption est maintenu immobile entre deux butées axiales du support coopérant avec le tampon d'absorption. Le tampon d'absorption est immobilisé par pincement axiale entre les butées axiales du support. On réduit ainsi le risque de frottement entre le tampon d'absorption et le support.
- Le support comporte deux parties emboîtées l'une dans l'autre, chaque partie définissant une butée axiale. Ce mode de réalisation est simple à mettre en oeuvre.
- Le dispositif comporte une forme d'expulsion de liquide résiduel située au voisinage immédiat et en regard de l'ouverture de distribution et portée par le capuchon de protection ou le support, lorsque le capuchon de protection est monté sur le dispositif. La forme d'expulsion expulse la majeure partie du liquide résiduel présente en aval de l'ouverture de distribution, notamment vers le tampon d'absorption de liquide résiduel disposé à proximité, c'est-à-dire que l'on évacue le liquide résiduel vers le tampon d'absorption. On comprend donc que le tampon d'absorption est disposé en aval de l'ouverture de distribution. On réalise ainsi un drainage de la majeure partie du liquide résiduel hors de l'ouverture de distribution. L'expulsion ou évacuation de liquide résiduel au voisinage de l'ouverture de distribution évite le développement de bactéries dans cette zone, ce qui est particulièrement intéressant lorsque le liquide distribué ne comporte pas d'agents conservateurs.
- Le dispositif peut prendre, lorsque le capuchon de protection est monté sur le dispositif, une configuration de fermeture hermétique du dispositif, avant sa première utilisation, dans laquelle le chemin d'évaporation du liquide résiduel est obturé entre le tampon d'absorption et l'extérieur du dispositif, et une configuration de ventilation du dispositif, dans laquelle le chemin d'évaporation est ouvert entre le tampon d'absorption et l'extérieur.
- Le dispositif comporte un chemin d'évaporation de liquide résiduel entre le tampon d'absorption et l'extérieur du dispositif lorsque le capuchon de protection est monté sur le dispositif, le capuchon de protection comprenant une enveloppe extérieure et une enveloppe intérieure, montées mobiles l'une par rapport à l'autre entre une première configuration, correspondant à une configuration de fermeture hermétique du dispositif, et une seconde configuration correspondant à une configuration de ventilation du dispositif, dans laquelle le chemin d'évaporation est ouvert entre le tampon d'absorption et l'extérieur. Ainsi, le capuchon de protection permet une ventilation telle que décrite dans le document FR 2 988 015, si bien que le liquide résiduel absorbé par le tampon d'absorption peut s'évaporer hors du dispositif.
- Le capuchon de protection comprend des orifices de passage d'air et un opercule obturant le chemin d'évaporation de liquide résiduel avant la première utilisation. L'opercule est amovible, de préférence jetable, et est rapporté sur le capuchon de protection. Lors de la première utilisation, l'utilisateur enlève l'opercule du dispositif qui passe de la configuration de fermeture hermétique à la configuration de ventilation.

La présente invention a également pour objet un procédé d'assemblage selon la revendication 14. Le tampon d'absorption étant préalablement assemblé au support de manière séparé, on minimise ainsi la génération de particules à l'intérieur du dispositif. En outre, on minimise également la génération de particules dans le dispositif lorsqu'on assemble l'ensemble tampon d'absorption et support sur le capuchon de protection. Enfin, l'étape supplémentaire optionnelle de soufflage permet avantageusement d'éliminer toute particule qui aurait pu être générée.

L'invention sera mieux comprise à la lecture des figures annexées, qui sont fournies à titre d'exemples et ne présentent aucun caractère limitatif, dans lesquelles :
- la figure 1 est une vue en coupe longitudinale d'un dispositif de distribution selon un premier mode de réalisation ;
- la figure 2 est une vue en perspective explosée de l'intérieur de l'enveloppe intérieure du capuchon de protection, du tampon d'absorption et du support, du dispositif de la figure 1 ;
- la figure 3 est une vue similaire à la figure 1 d'un dispositif selon un deuxième mode de réalisation ;
- la figure 4 est une vue en perspective explosée de l'intérieur de l'enveloppe intérieure du capuchon de protection, du tampon d'absorption et du support, du dispositif de la figure 3 ;
- la figure 5 est une vue similaire à la figure 1 d'un dispositif selon un troisième mode de réalisation en configuration de ventilation ;
- les figures 6 à 13 et 15 sont des vues similaires à la figure 1 d'un dispositif selon d'autres modes de réalisation ;
- la figure 14 est une vue en coupe illustrant le procédé d'assemblage du tampon, du support et du capuchon de protection du mode de réalisation présenté à la figure 13 ;
- la figure 16 est une vue en perspective du dispositif de la figure 15 en configuration de fermeture hermétique.
Seuls les modes de réalisation des Figures 11, 12, 15 et 16 sont selon l'invention.

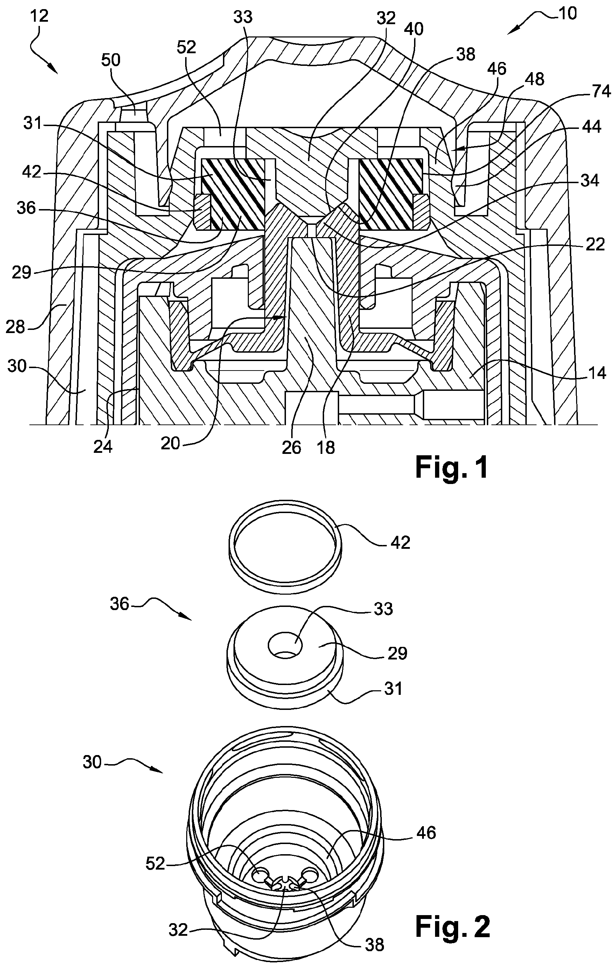

On a représenté sur les figures 1 et 2 un premier mode de réalisation d'un dispositif 10 de distribution de liquide. Le dispositif 10 est similaire à celui décrit dans la publication FR 2 988 015. Le dispositif 10 se trouve, sur la figure 1, en configuration de fermeture hermétique. Dans cet exemple non limitatif, le dispositif 10 est un dispositif de distribution de liquide sous forme de gouttes.

Le dispositif 10 comprend un capuchon de protection 12 et un embout de distribution 14 qui est destiné à être monté sur le col d'un réservoir (non représenté), par exemple par vissage. Ce réservoir est un réservoir de stockage de liquide ophtalmique. Le réservoir est déformable, de façon à distribuer du liquide par pression, de la part d'un utilisateur, sur le corps du réservoir, ce dernier pouvant présenter une certaine élasticité pour reprendre sa forme initiale après la pression exercée par l'utilisateur, ce qui génère une dépression à l'intérieur du réservoir.

L'embout de distribution 14 comprend une membrane 18, ou valve 18, formant une valve anti-retour, un support de valve 26 et une enveloppe supérieure 24. La valve 18 délimite, avec le support de valve 26, un canal 20 de distribution de liquide issu du réservoir. Ce canal 20 débouche sur une partie évasée, formant des moyens de formation de goutte 34, réalisée dans la valve 18 pour la formation de gouttes, définissant une ouverture de distribution 22 de liquide. La valve 18 est configurée de façon à pouvoir prendre une configuration de passage de liquide, au cours de laquelle du liquide peut atteindre l'ouverture de distribution 22, et une configuration de blocage du liquide, au cours de laquelle la valve 18 est plaquée contre l'extrémité supérieure du support de valve 26, bloquant ainsi le passage de liquide.

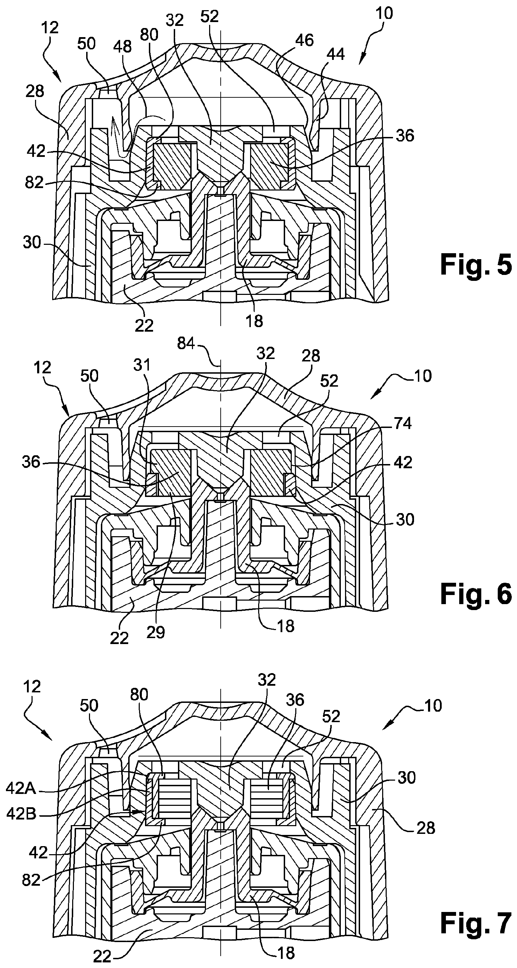

On va maintenant décrire le capuchon de protection 12, monté sur l'embout de distribution 14, de préférence par vissage. Le capuchon de protection 12 comprend, dans cet exemple, une enveloppe extérieure 28 et une enveloppe intérieure 30. Ces enveloppes intérieure et extérieure 30, 28 sont coaxiales, solidaires l'une de l'autre en étant montées mobiles l'une par rapport à l'autre en rotation et en translation longitudinale, entre une première configuration, correspondant à une configuration de fermeture hermétique du dispositif 10, visible sur la figure 1, et une seconde configuration correspondant à une configuration de ventilation du dispositif 10. Comme illustré sur la figure 5, dans la configuration de ventilation, un chemin d'évaporation 48 est ouvert entre le tampon d'absorption 36 décrit dans la suite et l'extérieur du dispositif 10, de façon que du liquide résiduel absorbé par ce tampon d'absorption 36 puisse s'évaporer. L'enveloppe intérieure 30 comprend en outre une forme d'expulsion 32 de liquide résiduel qui se trouverait dans ou au voisinage de l'ouverture de distribution 22. Cette forme d'expulsion 32 est située au voisinage immédiat et en regard de l'ouverture de distribution 22.

La forme d'expulsion 32 est également, dans cet exemple, une forme d'immobilisation de la valve 18 en position de blocage du liquide lorsque le capuchon de protection 12 est monté sur le dispositif 10. Par exemple, la forme d'expulsion 32 est un pion dont la forme générale est complémentaire des moyens de formation de goutte 34, réalisés dans la valve 18, par exemple la forme d'expulsion 32 est tronconique. Ainsi, la forme d'expulsion 32 assure une immobilisation de la valve 18 par pincement de cette dernière contre le support de valve 26.

Le capuchon de protection 12 comprend un tampon d'absorption 36 de liquide résiduel, fixé sur le capuchon de protection 12 par l'intermédiaire d'un support 42 lui-même fixé, dans cet exemple, sur l'enveloppe intérieure 30 du capuchon de protection 12.

Le tampon d'absorption 36 est de forme sensiblement annulaire. Il est disposé autour de la forme d'expulsion 32 du liquide résiduel. Il présente un orifice 33 recevant la forme d'expulsion 32 de l'enveloppe intérieure 30 du capuchon de protection 12. Cet orifice 33 a une forme générale cylindrique et peut être de diamètre variable. Il peut également être de forme tronconique et présenter à la surface d'éventuels reliefs et/ou creux. Par exemple, le tampon d'absorption 36 a un diamètre de 10 mm +/- 0,20 mm et l'orifice 33 disposé au centre a un diamètre de 4 mm +/- 0,15 mm.

La section de l'anneau du tampon d'absorption 36 est de forme sensiblement rectangulaire. Un des coins de la face intérieure du tampon d'absorption 36 pourrait éventuellement être biseauté et complémentaire de la forme de la valve 18. Avantageusement, les deux coins de la face intérieure pourraient être biseautés, le tampon d'absorption 36 peut donc être fixé sur le support 42 indifféremment dans un sens ou dans l'autre.

Le tampon d'absorption 36 peut comprendre une partie ou plusieurs parties de différentes formes et/ou dimensions. Dans l'exemple illustré dans la figure 1, le tampon d'absorption 36 est constitué de deux parties, une première partie 31 de forme générale annulaire, de plus grand diamètre qu'une deuxième partie 29, également de forme générale annulaire, entourée par le support 42.

De façon avantageuse, le tampon d'absorption 36 peut ne pas être en contact avec la valve 18 et ce afin d'éviter les contacts entre le liquide résiduel présent dans le tampon d'absorption 36 et la valve 18 lorsque le capuchon de protection 12 est monté sur le dispositif 10.

De façon également avantageuse, le tampon d'absorption 36 peut ne pas être en contact avec l'enveloppe intérieure 30 du capuchon de protection 12 et ce afin d'éviter les frottements entre le capuchon de protection 12 et le tampon d'absorption 36 responsables de la génération de particules. On comprend donc que le bord latéral 74 du tampon d'absorption 36 est espacé de contours de centrage du tampon d'absorption 36 porté par le support 42 dans le capuchon de protection 12, les moyens d'espacement du bord latéral du tampon d'absorption 36 sont portés par le support 42, par exemple par les parois latérales du support 42.

Le tampon d'absorption 36 comprend avantageusement un matériau poreux comportant un ou plusieurs matériaux hydrophiles. Le tampon d'absorption 36 peut également être un tampon multicouche, en combinaison ou en alternative avec le cas dans lequel il comprend un matériau poreux. Le matériau de tampon d'absorption 36 peut comprendre des matériaux tissés ou non tissés. Il peut par exemple comprendre du coton hydrophile, du polyéthylène (PE) ayant subi un traitement hydrophile, du polyéthylène téréphtalate (PET) ayant subi un traitement hydrophile, une mousse de poly(acétate de vinyle) (PVA) ou un mélange de plusieurs matériaux hydrophiles. Selon une variante particulièrement avantageuse, le tampon d'absorption 36 est réalisé dans un matériau comprenant de l'EVA (éthylène-acétate de vinyle).

Le support 42, sur lequel est fixé le tampon d'absorption 36, a une forme générale annulaire. Sa section est de forme sensiblement rectangulaire. Il peut être continu ou discontinu, et peut comprendre des reliefs ou creux qui permettraient par exemple de le fixer sur l'enveloppe intérieure 30 du capuchon de protection 12. De façon avantageuse, il est disposé sur le pourtour du tampon d'absorption 36 et se trouve en contact direct avec l'enveloppe intérieure 30 du capuchon de protection 12. Plus particulièrement dans l'exemple, il est positionné sur le pourtour de la deuxième partie 29 du tampon d'absorption 36, ce qui confère à l'ensemble un diamètre supérieur au diamètre de la première partie 31 du tampon d'absorption 36, permettant avantageusement de rentrer en contact avec l'enveloppe intérieure 30 du capuchon de protection 12 et évitant ainsi que le tampon d'absorption 36 pris dans son ensemble ne vienne frotter contre cette paroi de l'enveloppe intérieure 30. Le support 42 comprend avantageusement un matériau plus résistant que le matériau du tampon d'absorption 36. Le support 42 peut être réalisé en matière plastique, telle que du polyéthylène, traité ou non traité.

Le procédé d'assemblage du dispositif 10 va à présent être décrit.

On assemble tout d'abord le tampon d'absorption 36 et le support 42, de façon avantageuse par surmoulage ou par tout autre moyen de serrage mécanique. De cette façon, peu de particules, voire pas de particule du tout, sont générées dans le dispositif 10. Afin de retenir le tampon d'absorption 36 sur le support 42, on peut également envisager de poser le tampon d'absorption 36 sur le support 42.

Puis, on rapporte l'ensemble à l'intérieur du capuchon de protection 12. Plus précisément, on fixe le support 42 à l'intérieur de l'enveloppe intérieure 30, de manière à ce que le tampon d'absorption 36 se retrouve autour de la forme d'expulsion 32 de la valve 18 une fois le capuchon de protection 12 monté, sans être en contact avec les bords transversaux de l'enveloppe intérieure 30 du capuchon de protection 12. On vient ensuite monter l'enveloppe extérieure 28 sur l'enveloppe intérieure 30, par exemple par vissage, de façon que l'enveloppe extérieure 28 ne puisse plus être désolidarisée de l'enveloppe intérieure 30. On comprend que l'enveloppe extérieure 28 peut être assemblée avec l'enveloppe intérieure 30 avant l'insertion du support 42 et du tampon d'absorption 36. Avant de rapporter le tampon d'absorption 36 et le support 42 dans le capuchon de protection 12, on peut effectuer de façon particulièrement avantageuse une étape de soufflage d'air comprimé sur l'ensemble du tampon d'absorption 36 et du support 42, de manière à les nettoyer de toutes les particules qui auraient pu se déposer. Ensuite, le capuchon de protection 12 est prêt à être monté par vissage sur l'embout de distribution 14, puis une fois le réservoir rempli du liquide à distribuer, on vient visser l'ensemble formé par l'embout de distribution 14 et le capuchon de protection 12 sur le col du réservoir. Le dispositif 10 est prêt à être utilisé.

La première configuration, dite configuration de stockage ou de fermeture hermétique du dispositif 10 est représentée sur la figure 1, de sorte qu'il n'y a pas de communication possible entre le tampon d'absorption 36 et l'extérieur du dispositif 10. Dans cette configuration, il n'y a pas de communication entre des orifices de passage d'air 52 de l'enveloppe intérieure 30 et des orifices de passage d'air 50 de l'enveloppe extérieure 28. En effet, une couronne 44 de l'enveloppe extérieure 28 forme, avec une surface 46 de l'enveloppe intérieure 30, un moyen d'obturation du chemin d'évaporation 48. Dans ce mode de réalisation, cette surface 46 est conique et portée par une partie de l'enveloppe intérieure 30 qui forme également un contour de centrage du tampon d'absorption 36 dans le capuchon de protection 12, en l'occurrence dans l'enveloppe intérieure 30.

Lors de la première utilisation, l'utilisateur dévisse le capuchon de protection 12. Il serre l'enveloppe extérieure 28 dans une main et le réservoir dans l'autre main. Il fait tourner l'enveloppe extérieure 28 par rapport à l'enveloppe intérieure 30. Le mouvement de rotation appliqué par l'utilisateur au dispositif 10 génère un mouvement de translation longitudinal de l'enveloppe extérieure 28 par rapport à l'enveloppe intérieure 30, jusqu'à une configuration de ventilation. Dans cette configuration de ventilation, représentée sur la figure 5, grâce au déplacement longitudinal de l'enveloppe extérieure 28 par rapport à l'enveloppe intérieure 30, la couronne 44 de l'enveloppe extérieure 28 ne coopère plus avec la surface 46 de l'enveloppe intérieure 30, si bien qu'un espace se crée entre la couronne 44 et la surface 46, et donc le chemin d'évaporation 48 du liquide résiduel est ouvert entre le tampon d'absorption 36 et l'extérieur du dispositif 10.

Entre deux utilisations, l'utilisateur revisse le capuchon de protection 12 sur l'embout de distribution 14. Lorsque le capuchon 12 est monté sur l'embout de distribution 14, la forme d'expulsion 32 coopère avec la valve 18, en particulier avec les moyens de formation de goutte 34. En outre, cette forme d'expulsion 32 comporte des gorges 38 qui délimitent avec la valve 18 une voie d'évacuation 40 du liquide résiduel contenu dans les moyens de formation de goutte 34. Cette voie d'évacuation 40 permet d'évacuer le liquide résiduel vers le tampon d'absorption 36, dans lequel il est absorbé. Ensuite, le liquide absorbé par le tampon d'absorption 36 peut s'évaporer hors du dispositif 10 par le chemin d'évaporation 48 ouvert entre le tampon d'absorption 36 et l'extérieur du dispositif 10. En effet, l'enveloppe extérieure 28 et l'enveloppe intérieure 30 comportent chacune des moyens de verrouillage du dispositif 10 en configuration de ventilation, de sorte que lorsque l'utilisateur revisse le capuchon de protection 12 sur le dispositif 10, le chemin d'évaporation 48 reste ouvert entre le tampon d'absorption 36 et l'extérieur du dispositif 10.

Il n'y a donc pas de liquide résiduel qui stagne dans les moyens de formation de goutte 34 et on évite la formation de résidu solide du principe actif pouvant entraîner la délivrance d'une surdose lors de la délivrance d'une goutte ou la délivrance de particules solides en suspension dans la goutte lors de l'utilisation suivante du dispositif 10.

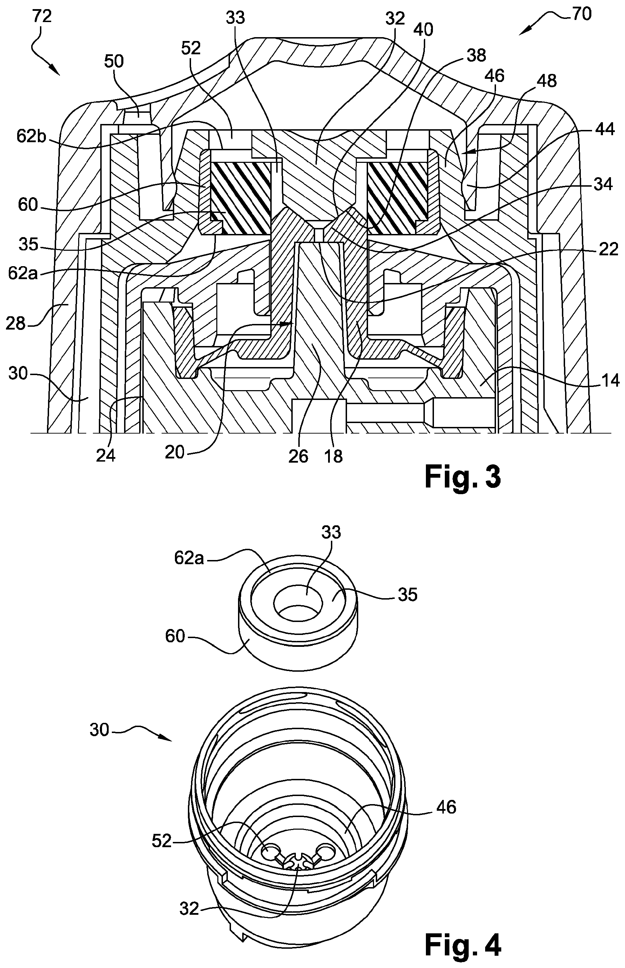

On a représenté sur les figures 3 et 4, un deuxième mode de réalisation du dispositif 70 de distribution de liquide, similaire au dispositif 10 de la figure 1. Dans ce qui suit, les éléments communs aux différents modes de réalisation sont identifiés par les mêmes références numériques. Ce dispositif 70 se trouve également en configuration de fermeture hermétique.

Dans cet exemple, le capuchon de protection 72 comprend un tampon d'absorption 35 en une seule partie et un support 60 sur lequel est fixé le tampon d'absorption 35. Le tampon d'absorption 35 est de forme générale annulaire de 10 mm +/- 0,20 mm de diamètre et présente un orifice 33 permettant le passage de la forme d'expulsion 32 de 4 mm +/- 0,15 mm de diamètre. Le support 60 s'étend, dans cet exemple, sur toute la hauteur du tampon d'absorption 35. Il présente en outre une partie 62 venant recouvrir au moins partiellement une face du tampon d'absorption 35. Dans cet exemple, une partie 62b du support 60, destinée à venir en butée contre le fond de l'enveloppe intérieure 30 du capuchon de protection 72, s'étend sur toute la face périphérique supérieure du tampon de protection 35. De cette manière avantageuse, le tampon d'absorption 35 ne rentre pas en contact avec l'enveloppe intérieure 30 et on évite ainsi tout frottement susceptible de générer des particules. La partie 62a du support 60, se trouvant du côté de la valve 18, recouvre partiellement la surface du tampon d'absorption 35 de manière à ce que la surface du tampon d'absorption 35, libre de support, puisse venir contre la valve 18 et jouer son rôle d'absorption du liquide résiduel présent sur la valve 18 après utilisation du dispositif 70 et remise du capuchon de protection 72.

Les figures 5 à 13 et 15 illustrent des modes de réalisations supplémentaires du dispositif 10. Dans ce qui suit, les éléments communs aux différents modes de réalisation sont identifiés par les mêmes références numériques que celles des figures 1 et 2.

Dans le mode de réalisation de la figure 5, le dispositif 10 est en configuration de ventilation de sorte que le chemin d'évaporation 48 du liquide résiduel est ouvert entre le tampon d'absorption 36 et l'extérieur du dispositif 10.

Le support 42 s'étend sur toute la hauteur du tampon d'absorption 36. Il présente en outre une butée axiale supérieure 80 et une butée axiale inférieure 82 qui, par coopération avec le tampon d'absorption 36, immobilisent le tampon d'absorption 36 dans le support 42.

On notera par ailleurs que la butée axiale supérieure 80 vient en butée dans l'enveloppe intérieure 30. Ainsi, lorsque l'on rapporte cet ensemble dans l'enveloppe intérieure 30, on enfonce le support 42 jusqu'à mise en butée dans l'enveloppe intérieure 30 de la butée axiale supérieure 80. On constate donc que le tampon d'absorption 36 ne rentre pas en contact avec l'enveloppe intérieure 30 tout en étant cependant immobilisé dans cette enveloppe intérieure 30.

Le support 42 peut être fixé sur le tampon d'absorption 36 par surmoulage du support 42.

Le mode de réalisation du dispositif 10 de la figure 6 est similaire au mode de réalisation de la figure 1. Il en diffère en ce que le tampon d'absorption 36 est posé sur le support 42. Il existe donc un espace entre le support 42 et la deuxième partie 29 du tampon d'absorption 36. Cependant, cet espace est tel que si le tampon d'absorption 36 bouge légèrement dans un plan perpendiculaire à l'axe central 84 du dispositif 10, le bord latéral 74 du tampon d'absorption 36 reste toujours espacé des parois internes de l'enveloppe intérieure 30.

On notera par ailleurs que le tampon d'absorption 36 est immobilisé axialement par pincement entre le support 42 et le fond de l'enveloppe intérieure 30.

Dans le mode de réalisation de la figure 7, le support 42 comporte une partie supérieure 42A et une partie inférieure 42B, emboîtées l'une dans l'autre, définissant respectivement les butées axiales supérieure 80 et inférieure 82. La butée axiale supérieure 80 vient en butée dans l'enveloppe intérieure 30. On constate également que le tampon d'absorption 36 est composé d'une pluralité de couches de tampon empilées l'une sur l'autre axialement.

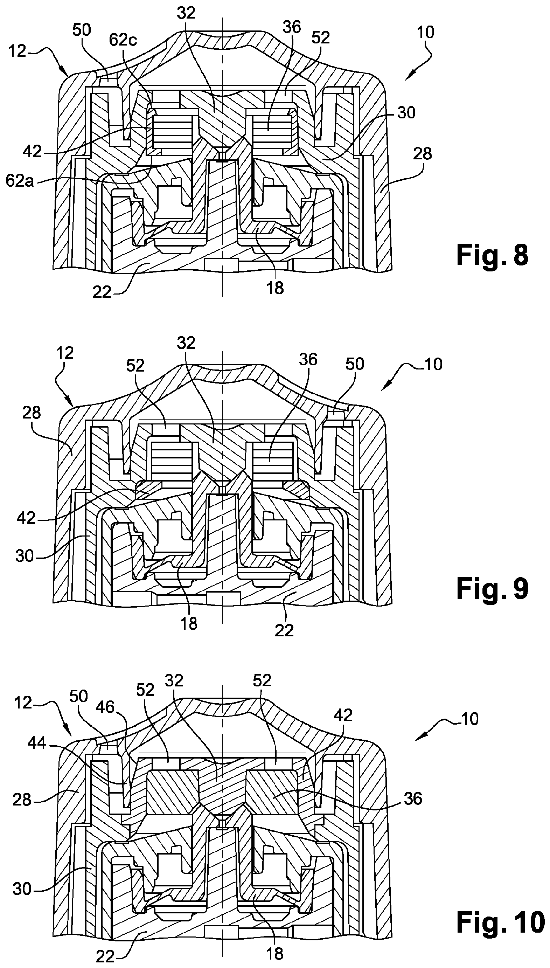

Le mode de réalisation de la figure 8 est similaire au mode de réalisation de la figure 3. Il en diffère en ce que le tampon d'absorption 36 est ici réalisé par un empilement axial de couches de tampon et en ce que le support 42 comprend une partie 62c se trouvant à l'opposé du côté de la valve 18 et recouvre partiellement la surface du tampon d'absorption 36 de manière à ce que les couches de tampon du tampon d'absorption 36 se trouvent axialement bloquées entre les parties 62a et 62c.

Le mode de réalisation de la figure 9 diffère des modes de réalisation précédents en ce que le support 42 ne forme pas un anneau autour du tampon d'absorption 36. Dans ce mode de réalisation, le support 42 forme un plateau sur lequel on vient poser le tampon d'absorption 36, dans le cas présent l'empilement de couches de tampon d'absorption. Le support 42 est ensuite encliqueté sur l'enveloppe intérieure 30 de sorte à fixer l'ensemble formé par le support 42 et le tampon d'absorption 36 dans l'enveloppe intérieure 30.

Dans le mode de réalisation de la figure 10, le support 42 porte la surface 46 qui coopère avec la couronne 44 de l'enveloppe extérieure 28 afin de fermer le chemin d'évaporation 48 du liquide résiduel en configuration de fermeture hermétique du dispositif 10. Le support 42 comprend également la forme d'expulsion 32 de liquide résiduel. Le bord latéral 74 du tampon d'absorption 36 ne vient donc pas en contact avec l'enveloppe intérieure 30 lorsque l'ensemble formé par le tampon d'absorption 36 et le support 42 sont rapportés dans le contour de centrage du tampon d'absorption 36 porté par l'enveloppe intérieure 30 du capuchon de protection 12. Il n'y a donc pas ou peu de formation de particules lorsque l'on rapporte le support 42 muni du tampon d'absorption 36 dans le capuchon de protection 12. On notera par ailleurs que le support 42 est rapporté par l'extérieur de l'enveloppe intérieure 28, c'est-à-dire par le haut.

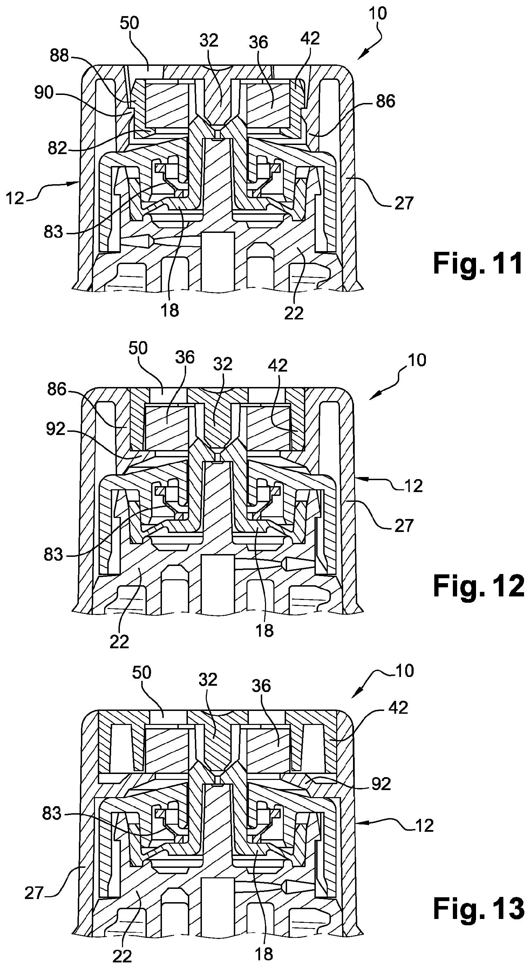

Les modes de réalisation des figures 11 à 16 représentent un dispositif 10 dont le capuchon de protection 12 ne comprend qu'une enveloppe unique 27 semblable à l'enveloppe intérieure 30 des modes de réalisation précédemment décrits et un élément ressort 83, élastiquement déformable, apte à solliciter la valve 18 vers sa position de blocage.

Dans le mode de réalisation de la figure 11, le capuchon de protection 12 comporte des orifices de passage d'air 50 du tampon d'absorption 36 vers l'extérieur du dispositif 10. On comprend donc que le dispositif 10 est représenté en configuration de ventilation. Le fond du capuchon de protection 12 comporte une couronne intérieure 86 qui forme le contour de centrage du tampon d'absorption 36 dans le capuchon de protection 12 ainsi que la forme d'expulsion 32 de liquide résiduel.

Le support 42 comporte une butée axiale inférieure 82 sur laquelle repose le tampon d'absorption 36. Le support 42 porte, sur sa surface radiale extérieure, des moyens d'encliquetage 88 qui coopèrent avec des moyens d'encliquetage complémentaires 90 portés par la couronne intérieure 86 de sorte à fixer l'ensemble formé par le support 42 et le tampon d'absorption 36 dans le capuchon de protection 12.

Dans les modes de réalisation des figures 12, 13 et 15, le support 42 comporte les orifices de passage d'air 50 du tampon d'absorption 36 vers l'extérieur du dispositif 10 ainsi que la forme d'expulsion 32 de liquide résiduel.

Dans les modes de réalisation des figures 12 et 13, le support 42 est rapporté dans le capuchon de protection 12 par l'extérieur, c'est-à-dire par le haut, du capuchon de protection 12, contrairement aux modes de réalisation des figures 1 à 9, 11 et 15.

Dans ces modes de réalisation, le support 42 est fixé dans le capuchon de protection 12 par serrage mécanique. Par ailleurs, on notera que le capuchon de protection 12 comporte une butée axiale 92 qui permet le positionnement du support 42 par rapport au capuchon de protection 12.

Dans le mode de réalisation de la figure 12, le contour de centrage du tampon d'absorption 36 dans le capuchon de protection 12 est formé par la couronne intérieure 86 alors que dans le mode de réalisation de la figure 13, le contour de centrage du tampon d'absorption 36 dans le capuchon de protection 12 est formé par la paroi latérale du capuchon de protection 12.

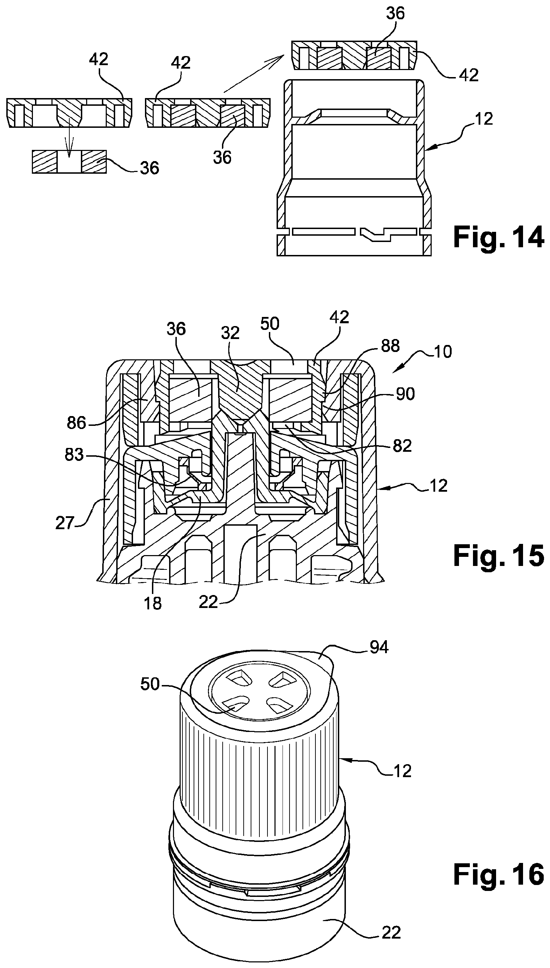

La figure 14 illustre les différentes étapes du procédé d'assemblage du tampon d'absorption 36 et du support 42 avec le capuchon de protection 12. A la différence de ce qui a été décrit précédemment, on voit que l'ensemble formé du tampon d'absorption 36 et du support 42 est rapporté par le haut du capuchon de protection 12.

Dans le mode de réalisation de la figure 15, le support 42 est rapporté par l'intérieur du capuchon de protection 12 et y est fixé par encliquetage, par coopération de moyens d'encliquetage 88, 90 respectivement portés par le support 42 et la couronne interne 86 du capuchon de protection 12. Le support 42 comporte en outre une pluralité de butées axiales inférieures 82 qui sont passées en force lors de l'insertion du tampon d'absorption 36 dans le support 42.

La figure 16 illustre le dispositif 10 de la figure 15 en configuration de fermeture hermétique du dispositif 10 avant la première utilisation. Le capuchon de protection 12 comprend un opercule 94 obturant les orifices de passage d'air 50 et donc le chemin d'évaporation 48 de liquide résiduel avant la première utilisation. L'opercule 94 est amovible, de préférence jetable, et est rapporté sur le capuchon de protection 12. Lors de la première utilisation, l'utilisateur enlève l'opercule 94 du dispositif 10 qui passe de la configuration de fermeture hermétique à la configuration de ventilation.

On comprend que l'invention n'est pas limitée aux exemples présentés ci-dessus et que des modifications apparaîtront clairement à l'homme du métier dans la portée des revendications ci-jointes.

## Revendications

1. Dispositif de distribution de liquide ophtalmique sous forme de gouttes (10), comprenant un capuchon de protection (12) d'une ouverture de distribution de liquide (22), le capuchon de protection (12) étant muni d'un tampon d'absorption (36) de liquide résiduel, réalisé dans un matériau absorbant, le fond du capuchon de protection (12) comportant une couronne intérieure (86), le tampon d'absorption (36) étant fixé sur un support (42) réalisé dans un matériau plus résistant que le tampon d'absorption (36), **caractérisé en ce que** ce support (42) est par ailleurs fixé dans le capuchon de protection (12) par assemblage mécanique, par exemple par serrage, encliquetage ou collage, de sorte que le support (42) soit bloqué par rapport au capuchon de protection (12), et **en ce que** le support (42) porte des moyens d'encliquetage (88) qui coopèrent avec des moyens d'encliquetage complémentaires (90) portés par la couronne intérieure (86).

2. Dispositif (10) selon la revendication précédente, dans lequel le support (42) est réalisé en matière plastique, telle que du polyéthylène.

3. Dispositif (10) selon l'une quelconque des revendications précédentes, dans lequel le support (42) a une forme annulaire.

4. Dispositif (10) selon l'une quelconque des revendications précédentes, dans lequel le support (42) s'étend sur toute la hauteur du tampon d'absorption.

5. Dispositif (10) selon l'une quelconque des revendications précédentes, dans lequel le tampon d'absorption (36) est fixé sur le support (42) par surmoulage.

6. Dispositif (10) selon l'une quelconque des revendications précédentes, dans lequel le tampon d'absorption (36) est réalisé dans un matériau poreux.

7. Dispositif (10) selon l'une quelconque des revendications précédentes, dans lequel le tampon d'absorption (36) est réalisé dans un matériau comprenant de l'EVA (éthylène-acétate de vinyle).

8. Dispositif (10) selon l'une quelconque des revendications précédentes, dans lequel le tampon d'absorption (36) comprend au moins deux couches de tampon empilées.

9. Dispositif (10) selon l'une quelconque des revendications précédentes, dans lequel le tampon d'absorption (36) est maintenu immobile entre deux butées axiales (80, 82) du support (42) coopérant avec le tampon d'absorption (36).

10. Dispositif (10) selon la revendication précédente, dans lequel le support (42) comporte deux parties emboîtées l'une dans l'autre, chaque partie définissant une butée axiale (80, 82).

11. Dispositif (10) selon l'une quelconque des revendications précédentes, comportant une forme d'expulsion (32) de liquide résiduel située au voisinage immédiat et en regard de l'ouverture de distribution (22) et portée par le capuchon de protection (12) ou le support (42), lorsque le capuchon de protection (12) est monté sur le dispositif (10).

12. Dispositif (10) selon l'une quelconque des revendications précédentes, pouvant prendre, lorsque le capuchon de protection (12) est monté sur le dispositif (10), une configuration de fermeture hermétique du dispositif (10), avant sa première utilisation, dans laquelle un chemin d'évaporation du liquide résiduel est obturé entre le tampon d'absorption (36) et l'extérieur du dispositif (10), et une configuration de ventilation du dispositif (10), dans laquelle le chemin d'évaporation est ouvert entre le tampon d'absorption (36) et l'extérieur.

13. Dispositif (10) selon la revendication 12, dans lequel le capuchon de protection (12) comprend des orifices de passage d'air (50) et un opercule (94) obturant le chemin d'évaporation (48) de liquide résiduel avant la première utilisation.

14. Procédé d'assemblage d'un dispositif (10) selon l'une quelconque des revendications précédentes, au cours duquel on assemble tout d'abord le tampon d'absorption (36) et le support (42), puis on rapporte l'ensemble à l'intérieur du capuchon de protection (12), le procédé comprenant de préférence en outre une étape de soufflage d'air comprimé sur l'ensemble du tampon d'absorption (36) et du support (42), avant de les rapporter dans le capuchon de protection (12) par assemblage mécanique, par exemple par serrage, encliquetage ou collage, de sorte que le support (42) soit bloqué par rapport au capuchon de protection (12).

## Patentansprüche

1. Ausgabevorrichtung für tropfenförmige ophthalmische Flüssigkeit (10), umfassend eine Schutzkappe (12) für eine Flüssigkeitsausgabeöffnung (22), wobei die Schutzkappe (12) mit einem Absorptionskissen (36) für Restflüssigkeit versehen ist, das aus einem absorbierenden Material gefertigt ist, der Boden der Schutzkappe (12) umfassend einen inneren Kranz (86), wobei das Absorptionskissen (36) an einem Halter (42) befestigt ist, der aus einem stärkeren Material als das Absorptionskissen (36) gefertigt ist, **dadurch gekennzeichnet, dass** dieser Halter (42) ferner durch mechanische Zusammenfügen, beispielsweise durch Klemmen, Einrasten oder Kleben, in der Schutzkappe (12) befestigt ist, sodass der Halter (42) in Bezug auf die Schutzkappe (12) arretiert ist, und dass der Halter (42) Einrasteinrichtungen (88) trägt, die mit komplementären Einrasteinrichtungen (90) zusammenwirken, die von dem inneren Kranz (86) getragen werden.

2. Vorrichtung (10) nach dem vorherigen Anspruch, wobei der Halter (42) aus Kunststoff, wie beispielsweise Polyethylen, gefertigt ist.

3. Vorrichtung (10) nach einem der vorherigen Ansprüche, wobei der Halter (42) eine ringförmige Form aufweist.

4. Vorrichtung (10) nach einem der vorherigen Ansprüche, wobei sich der Halter (42) über die gesamte Höhe des Absorptionskissens erstreckt.

5. Vorrichtung (10) nach einem der vorherigen Ansprüche, wobei das Absorptionskissen (36) durch Umspritzen an dem Halter (42) befestigt ist.

6. Vorrichtung (10) nach einem der vorherigen Ansprüche, wobei das Absorptionskissen (36) aus einem porösen Material gefertigt ist.

7. Vorrichtung (10) nach einem der vorherigen Ansprüche, wobei das Absorptionskissen (36) aus einem Material gefertigt ist, das EVA (Ethylen-Vinylacetat) umfasst.

8. Vorrichtung (10) nach einem der vorherigen Ansprüche, wobei das Absorptionskissen (36) mindestens zwei gestapelte Kissenschichten umfasst.

9. Vorrichtung (10) nach einem der vorherigen Ansprüche, wobei das Absorptionskissen (36) unbeweglich zwischen zwei axialen Anschlägen (80, 82) des mit dem Absorptionskissen (36) zusammenwirkenden Halters (42) gehalten wird.

10. Vorrichtung (10) nach dem vorherigen Anspruch, wobei der Halter (42) zwei ineinander geschachtelte Teile umfasst, wobei jedes Teil einen axialen Anschlag (80, 82) definiert.

11. Vorrichtung (10) nach einem der vorherigen Ansprüche, umfassend eine Ausstoßform (32) für Restflüssigkeit, die sich in unmittelbarer Nähe und gegenüber der Ausgabeöffnung (22) befindet und von der Schutzkappe (12) oder dem Halter (42) getragen wird, wenn die Schutzkappe (12) an der Vorrichtung (10) montiert ist.

12. Vorrichtung (10) nach einem der vorherigen Ansprüche, die, wenn die Schutzkappe (12) an der Vorrichtung (10) montiert ist, eine Konfiguration zum hermetischen Verschließen der Vorrichtung (10) vor ihrer ersten Verwendung annehmen kann, wobei ein Verdampfungsweg der Restflüssigkeit zwischen dem Absorptionskissen (36) und der Außenseite der Vorrichtung (10) verschlossen ist, und eine Lüftungskonfiguration der Vorrichtung (10), wobei der Verdampfungsweg zwischen dem Absorptionskissen (36) und der Außenseite offen ist.

13. Vorrichtung (10) nach Anspruch 12, wobei die Schutzkappe (12) Luftdurchlassöffnungen (50) und einen Deckel (94) umfasst, der den Verdampfungsweg (48) von Restflüssigkeit vor der ersten Verwendung verschließt.

14. Verfahren zum Zusammenbau einer Vorrichtung (10) nach einem der vorherigen Ansprüche, wobei zuerst das Absorptionskissen (36) und der Halter (42) zusammengebaut werden und dann das Ganze in das Innere der Schutzkappe (12) zurückgebracht wird, wobei das Verfahren vorzugsweise zusätzlich einen Schritt umfasst, wobei das gesamte Absorptionskissen (36) und der Halter (42) mit Druckluft angeblasen werden, bevor sie durch mechanisches Zusammenfügen, beispielsweise durch Klemmen, Einrasten oder Kleben, in die Schutzkappe (12) zurückgebracht werden, sodass der Halter (42) in Bezug auf die Schutzkappe (12) blockiert wird.

## Claims

1. A drop-shaped ophthalmic liquid dispensing device (10) comprising a protective cap (12) for a liquid dispensing opening (22), the protective cap (12) being provided with a residual liquid absorption pad (36) made of an absorbent material, the bottom of the protective cap (12) having an inner rim (86), the absorption pad (36) being fixed to a support (42) made of a material which is stronger than the absorption pad (36), **characterised in that** this support (42) is also fixed in the protective cap (12) by mechanical assembly, for example by clamping, snap-fastening or adhesive bonding, so that the support (42) is locked relative to the protective cap (12), and **in that** the support (42) carries snap-fastening means (88) which cooperate with complementary snap-fastening means (90) carried by the inner rim (86).

2. A device (10) according to the preceding claim, in which the support (42) is made of plastic, such as polyethylene.

3. A device (10) according to any one of the preceding claims, in which the support (42) has an annular shape.

4. A device (10) according to any one of the preceding claims, in which the support (42) extends over the entire height of the absorption pad.

5. A device (10) according to any one of the preceding claims, in which the absorption pad (36) is fixed to the support (42) by overmoulding.

6. A device (10) according to any one of the preceding claims, in which the absorption pad (36) is made of a porous material.

7. A device (10) according to any one of the preceding claims, wherein the absorption pad (36) is made of a material comprising EVA (ethylene vinyl acetate).

8. A device (10) according to any one of the preceding claims, wherein the absorption pad (36) comprises at least two stacked pad layers.

9. A device (10) according to any one of the preceding claims, in which the absorption pad (36) is held immobile between two axial stops (80, 82) of the support (42) cooperating with the absorption pad (36).

10. A device (10) according to the preceding claim, in which the support (42) comprises two interlocking parts, each part defining an axial stop (80, 82).

11. A device (10) according to any one of the preceding claims, comprising a residual liquid expulsion form (32) located in the immediate vicinity of and facing the dispensing opening (22) and carried by the protective cap (12) or the support (42), when the protective cap (12) is mounted on the device (10).

12. A device (10) according to any one of the preceding claims, capable of assuming, when the protective cap (12) is mounted on the device (10), a configuration of hermetic closure of the device (10), before its first use, in which an evaporation path of residual liquid is closed off between the absorption pad (36) and the outside of the device (10), and a configuration of ventilation of the device (10), in which the evaporation path is open between the absorption pad (36) and the outside.

13. A device (10) according to claim 12, in which the protective cap (12) comprises air passage orifices (50) and a seal (94) closing off the evaporation path (48) of residual liquid before the first use.

14. A method of assembling a device (10) according to any one of the preceding claims, during which the absorption pad (36) and the support (42) are first assembled, and then the assembly is brought inside the protective cap (12), the method preferably comprising a step of blowing compressed air onto the assembly of absorption pad (36) and support (42), before they are brought into the protective cap (12) by mechanical assembly, for example by clamping, snap-fastening or adhesive bonding, so that the support (42) is locked relative to the protective cap (12).
